# EUROPEAN PATENT SPECIFICATION

(11) **EP 2 533 791 B1**
(45) Date of publication and mention of the grant of the patent: **25.01.2017**
(21) Application number: 10842737.8
(22) Date of filing: 21.12.2010
(51) Int. Cl.: A61K 38/00

(54) **LEUCINE/PEPTIDE COMPOSITION AND METHOD OF FORMULATION**
LEUCIN-/PEPTIDZUSAMMENSETZUNG UND VERFAHREN ZUR FORMULIERUNG
COMPOSITION LEUCINE/PEPTIDE ET SON PROCÉDÉ DE FORMULATION

(30) Priority: 21.12.2009 US 288767 P
(43) Date of publication of application: 19.12.2012
(73) Proprietor: Glanbia Nutritionals (Ireland) Ltd., Kilkenny (IE)
(72) Inventor: PETERSEN, Brent, Twin Falls Idaho 83301 (US); WARD, Loren, Twin Falls Idaho 83301 (US); BASTIAN, Eric, Twin Falls Idaho 83301 (US)
(74) Representative: Kelly, Donal Morgan
(86) International application number: PCT/US2010/061624
(87) International publication number: WO 2011/084816

(56) References cited:
- WO-A1-2007/095733
- US-A- 4 091 120
- US-A1- 2001 049 361
- US-A1- 2007 128 252
- US-A1- 2009 042 770
- US-A1- 2009 042 770
- US-A1- 2009 203 789
- US-A1- 2009 203 789
- US-B1- 6 528 067

## Description

### Cross-Reference to Related Applications

This application claims the benefit of priority of U.S. Provisional Patent Application Number 61/288,767, filed December 21, 2009.

### Field of the Invention

The present invention relates to compositions and methods for increasing amino acid solubility. More specifically, the invention relates to methods for formulating products comprising amino acids in a more soluble form and compositions made by such methods.

### Background of the Invention

Leucine is a branched-chain amino acid and an essential amino acid. It is the only amino acid that is converted to acetyl-coenzyme A and alpha-ketoacid, and it is an important source of nitrogen for synthesis of glutamine. In addition to impacting protein synthesis and degradation, leucine also stimulates glucose uptake by protein kinase C (PKC), while insulin modulates glucose uptake via protein kinase B.

Loss of muscle tissue often occurs as a result of aging, malnutrition, and catabolic disease, such as burns, sepsis, and cancer. Dietary protein supplementation may be beneficial, but supplementation with the essential amino acid leucine has been shown to be especially beneficial. Dietary leucine has, for example, been shown to suppress the rate of myofibrillar protein degradation and muscle weight loss in rats. Leucine also stimulates muscle protein synthesis and modulates the activity of various proteins involved in the control of mRNA translation. Leucine may stimulate protein synthesis directly or through its metabolite, alpha-ketoisocaproic acid. Leucine may stimulate translation either independently or by interaction with the mammalian target of rapamycin (mTOR).

Formulating compositions comprising leucine, however, is often made more difficult by the fact that leucine, one of several hydrophobic amino acids, is not very soluble in water (about 0.08 moles/liter). Various attempts have been made to form compositions comprising leucine in a more soluble form, including forming peptides comprising leucine in conjunction with more hydrophilic amino acids which are more soluble, as well as forming complexes of leucine and more soluble moieties such as nitrate to form the more soluble leucine nitrate (Kramer, R., et al., US20090076110A1). It is beneficial, however, to provide leucine in its free amino acid form and it would therefore be of great benefit to develop a method for forming compositions comprising free leucine while increasing the solubility of leucine when added to an aqueous composition. Such a method may also be of benefit for producing more soluble forms of other amino acids, as well.

### Summary of the Invention

The present invention relates to a method for making a composition, the method comprising admixing at least one micronized amino acid with at least one protein to produce a solids mixture comprising the at amino acid and the protein, forming an aqueous suspension comprising the solids mixture, and drying the-suspension to provide a protein/amino acid product, the product providing the amino acid in a more soluble form than that of the free amino acid alone. The invention also relates to a method comprising micronizing at least one amino acid so that an amino acid product is formed wherein at least about 90% of the amino acid product comprises particle sizes of less than about 160 microns; admixing the amino acid product with at least one protein so that the amino acid product comprises from about 5 to about 90 percent of a solids mixture formed by the amino acid product and the at least one protein; admixing the solids mixture with water to form a suspension wherein the solids mixture comprises from about 1 percent to about 55 percent of the suspension; blending the suspension to form a substantially homogeneous suspension; and stirring the suspension during drying to maintain the homogeneous suspension during drying. Some aspects of the invention provide L-leucine as the at least one amino acid, and in other aspects the at least one amino acid may be any of the amino acids, including, for example, hydrophobic amino acids such as the branched-chain amino acids. In some aspects the at least one protein comprises one or more whole proteins, one or more protein fragments, and/or one or more peptides. In some aspects, the at least one protein is derived from whey protein. In some aspects of the method, the solids mixture comprises from about 7 percent to about 55 percent of the suspension. In some aspects, the solids mixture comprises about 15 to about 20 percent of the suspension.

The invention also relates to compositions made by the method, the compositions comprising amino acids in a more soluble form. The inventors have also found that these compositions, having lower viscosity, are more amenable to heat-treatment at higher temperatures without the undesirable increase in viscosity that results in other protein compositions having similar solids content.

In some aspects, a defoamer is added during the step of blending the suspension and the blending is performed with shear. In some aspects, the amino acid product is micronized to form particle sizes of from about 7 to about 10 microns.

### Brief Description of the Drawings

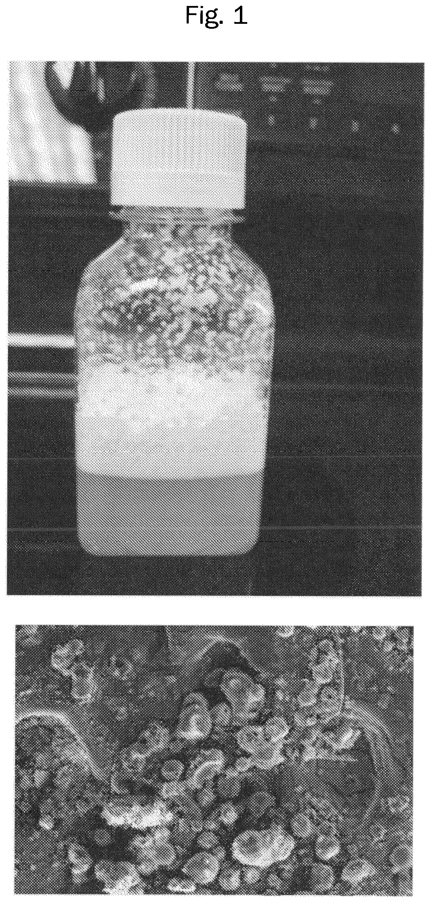

Fig. 1 illustrates the results of admixing amino acid and protein in the absence of the method of the invention. The top photograph shows the resulting mixture after being admixed with water. The bottom photograph is a scanning electron micrograph of the particles of the mixture.

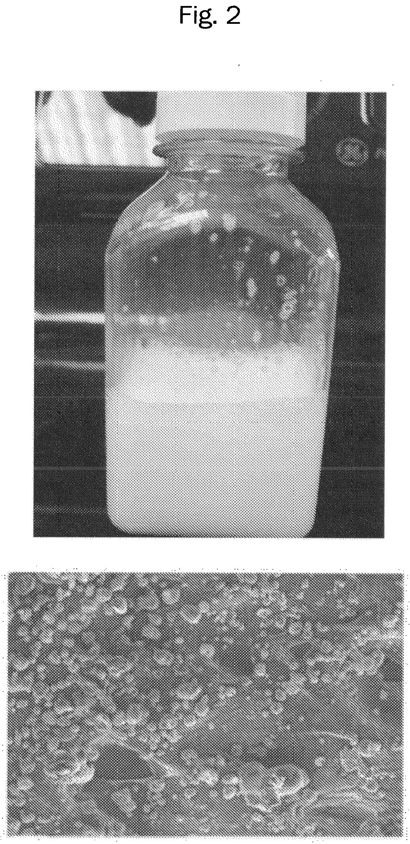

Fig. 2 illustrates the results of admixing amino acid and protein using the method of the invention. The top photograph shows the resulting mixture after being admixed with water. The bottom photograph is a scanning electron micrograph of the particles of the mixture.

### Detailed Description

The inventors have discovered that co-processing at least one micronized hydrophobic amino acid, such as, for example, leucine, isoleucine, valine, arginine tryptophan, or combinations thereof, with at least one protein by admixing the at least one amino acid with the at least one protein, suspending them in suspension, and then co-drying them, produces a composition that provides the amino acid and protein in a more soluble, less viscous form while still retaining the benefits of free amino acid in the composition. Preferably, the suspension is maintained in a substantially homogeneous suspension as far as is practicable during the drying process. It is also beneficial to the outcome of the process for the particle size of the at least one amino acid to be of less than about 160 microns, and more preferably less than about 20 microns. By way of example, leucine may be incorporated into a composition of the invention with excellent results at a micronization level of from about 7 to about 10 microns.

As used herein, "at least one amino acid" is a composition of one or more amino acids generally provided commercially in a substantially solid form, such as a powder. However, since the at least one amino acid will be admixed with at least one protein and suspended in suspension, it is within the scope of the invention to utilize micronized amino acids in liquid, as well as those provided in solid form. These are often referred to as "free" amino acids by those of skill in the art. In various aspects, the amino acid may comprise a branched-chain amino acid such as, for example, leucine, isoleucine, and/or valine, as well as other amino acids, particularly those amino acids. Hydrophobic amino acids, for example, have typically been difficult to formulate in liquid compositions, and this is made much easier by preparing amino acid compositions according to the method of the invention. "At least one protein," as used herein, is a composition of one or more proteins, protein fragments, and/or peptides in a substantially solid form, such as, for example, a powder. However, since the at least one protein will be admixed with at least one amino acid and suspended in suspension, it is within the scope of the invention to utilize protein in liquid, as well as in solid form.

In the method of the invention, at least one amino acid comprising at least about 90% particles of less than about 160 microns is admixed with at least one protein so that the at least one amino acid comprises from about 5 to about 90 percent of a solids mixture formed by the amino acid and the protein. The at least one protein may comprise synthesized proteins and/or peptides, but it may be advantageous and more economical to utilize isolated proteins, protein fragments, isolated peptides, and/or peptides obtained by hydrolyzing proteins. Proteins may be derived from milk, such as, for example, whey protein, whey protein concentrate, and/or whey protein isolate from bovine milk, and/or may be selected from the group consisting of soy, egg, vegetable, fish, wheat, rice, corn, fungal proteins, meat or other protein sources known to those of skill in the art, and combinations thereof. Whey protein concentrates, for example, are commercially available from a variety of sources, such as, for example, Avonlac™ from Glanbia Nutritionals, Monroe, Wisconsin. Whey protein isolates may be obtained by methods known to those of skill in the art or may be purchased from commercial sources such as, for example, Glanbia Nutritionals, which produces Provon^{®}. Milk protein concentrates and milk protein isolates such as the Solmiko^{®} products from Glanbia Nutritionals, as well as Glanbia's BarGain^{®}, which is a source of both whey and soy protein, also provide proteins which may be used to produce peptides for the composition and method of the invention.

The solids mixture is admixed with water to form a suspension wherein the solids mixture comprises from about 1 percent to about 55 percent of the suspension. For L-leucine, for example, a solids mixture comprising approximately 17 percent of the suspension provides excellent results, as does a suspension comprising about 55 percent solids. Therefore, in various aspects, the range of the solids mixture may comprise sub-ranges within the 1 percent to 55 percent range, such as from about 7 percent to about 55 percent of the suspension and from about 15 to about 20 percent of the suspension. The suspension is blended to provide a substantially homogeneous suspension (i.e., as homogeneous as is practicable under standard processing conditions known to those of skill in the art). Blending/mixing may be accomplished by a variety of means, including but not limited to the use of one or more homogenizers, centrifugal pumps, high shear mixers, microfluidizers, static mixers, and agitator tanks or a combination thereof. The suspension is then dried, with the homogeneity of the suspension being maintained as much as possible during the drying process, to produce a final product that forms a dry powder.

To provide a substantially homogeneous suspension, it is advisable to blend the suspension with shear and to add a defoamer. Optionally, a pasteurization step may be performed following blending. For the step of drying and by way of a non-limiting example, the following conditions may be used to achieve a good yield: 225°F inlet temperature, 85°F outlet temperature, pressure 3 bars (43.5 psi). Suitable equipment for drying may be obtained from a variety of commercial sources and such equipment may readily be identified by those of skill in the art. Drying conditions may be altered by those of skill in the art. Procedures for drying suspensions are described, for example, in the Handbook of Industrial Drying (Mujumdar, A.S., CRC Press (Taylor and Francis Group), Boca Raton, Florida, 2007). Suitable mixers include, for example, high shear batch mixers commercially available from a variety of sources such as Silverson Machines, Inc., East Longmeadow, Massachusetts. For example, a mixer such as the Silverson Flashblend^{®} mixer, which is adapted for mixing powders with liquid and forming homogeneous suspensions thereof, may be used.

Micronizing the at least one amino acid may be performed using a Micronizer Jet Mill (Sturtevant, Inc., Hanover, Massachusetts), for example. Suitable micronizers are available from a variety of commercial sources and may be chosen by those of skill in the art for the particular conditions and batch sizes desired.

The method of the invention is especially useful for producing more soluble forms of branched-chain amino acids such as leucine (L-leucine), isoleucine, and valine. Where it is desirable that the at least one protein comprise peptides having a higher concentration of leucine, as well, these may be obtained using the method described in United States Patent Application Publication Number US20090264363A1 (Ward, L. et al.), which may be used to prepare peptides for use in the method of the present invention. Using such peptides, it is therefore possible to produce a leucine supplement comprising a combination of free L-leucine amino acid and leucine-containing peptides which produces a more soluble form for the delivery of L-leucine. Compositions made by the method of the invention may be used in, for example, ready-to-mix formulations comprising amino acids and/or amino acids blended with other ingredients, in ready-to-drink formulations comprising amino acids and/or amino acids blended with other ingredients, in pills, in tablets, in bars, in shots, in gels, in protein balls, and/or in bakery items.

While not intending to be bound by theory, the inventors believe that co-processing the micronized at least one amino acid with at least one protein as described by the method of the invention, creates, or possibly interferes with, intermolecular non-covalent associations and alter the solubility of the resulting mixture (and therefore the amino acid, as compared to that of the corresponding free amino acid), as well as producing a thinning effect and decreasing the viscosity of the protein(s) in suspension. Heat treatment of proteins, which may be required for processes such as pasteurization, has been suggested to alter disulfide bonds in proteins and to increase viscosity of the proteins in suspension. Processing the at least one amino acid and the at least one protein as provided by the method of the invention results in a product which may be used to formulate liquid compositions having higher solids content, yet which still may be heat-treated without becoming undesirably viscous.

Amino acid interactions with and within proteins have been described previously. For example, amino acid composition of proteins has been reported to affect disulfide bond formation, with weakly hydrophilic and aromatic amino acids being more common in the areas near disulfide bonds, while aliphatic and hydrophobic residues are less common Marques, J.F.R. et al., "Amino Acid Patterns around Disulfide Bonds," Int. J. Mol. Sci. 2010, 11, 4673-4686). Arginine, a hydrophilic amino acid, weakens hydrophobic interactions between IL-6 and phenyl-sepharose, improving protein elution in hydrophobic interaction chromatography (Tsumoto, K., et al., "Arginine improves protein elution in hydrophobic interaction chromatography: The cases of human interleukin-6 and activin-A." Journal of Chromatographs A, 22 June 2007, Vol. 1154 (1-2): 81-86). According to Ashokkumar et *al*., "[i]n the dairy industry, the stability of dairy proteins toward heat treatment is a major processing issue. Exposure of whey proteins to temperatures in excess of 70°C causes denaturation, which in turn leads to protein aggregation through both hydrophobic interactions and the formation of intermolecular disulfide bonds" ("Sonication increases the heat stability of whey proteins," J. Dairy Sci. 92 :5353-5356). Ashokkumar et *al*. utilize a combination of heat treatment and sonication to disrupt these aggregates. Utilizing the method of the present invention, however, protein viscosity can be decreased, even with high solids content, with the added benefit of providing one or more free amino acids in a more soluble form.

Those of skill in the art, given the disclosure herein, may find multiple additional uses for products of the invention, as well.

The invention may be further described by means of the following non-limiting examples.

### Example

L-leucine (136 grams) was combined with 204 grams of whey protein isolate (Glanbia Nutritionals, Inc., Twin Falls, Idaho) according to the method previously described. A stable 10% suspension was formed with the resulting product, demonstrating the improved solubility of the amino acid composition produced by the method of the invention.

## Claims

1. A method for producing an amino acid composition with increased solubility, the method comprising admixing at least one micronized amino acid, comprising at least about 90 percent particles of less than about 160 microns, with at least one protein to produce a solids mixture comprising the amino acid and protein, suspending the solids mixture to form a substantially homogeneous aqueous suspension, and drying the suspension to produce an amino acid/protein product.

2. The method of claim 1 wherein the micronized amino acid has a particle size of from about 7 to about 10 microns.

3. The method of claim 1 wherein the amino acid is a hydrophobic amino acid.

4. The method of claim 1 wherein the amino acid is a branched-chain amino acid.

5. The method of claim 1 wherein the protein comprises peptides derived from whey protein.

6. The method of claim 1 wherein the solids mixture comprises from about 7 to about 55 percent of the suspension.

7. The method of claim 1 wherein the amino acid comprises from about 5 to about 90 percent of the solids mixture.

8. An amino acid composition with improved solubility of at least one amino acid, the composition made by a method comprising co-processing a mixture comprising at least one micronized amino acid, comprising at least about 90 percent particles of less than about 160 microns, and at least one protein to produce a substantially homogeneous aqueous suspension; and drying the suspension to give a substantially solid and/or powdered peptide/amino acid product.

9. The amino acid composition of claim 8 wherein the micronized amino acid has a particle size of from about 7 to about 10 microns.

10. The amino acid composition of claim 8 wherein the amino acid is a hydrophobic amino acid.

11. The amino acid composition of claim 8 wherein the amino acid is a branched-chain amino acid.

12. The amino acid composition of claim 8 wherein the protein comprises peptides derived from whey protein.

13. The amino acid composition of claim 8 wherein the solids mixture comprises from about 7 to about 55 percent of the suspension.

14. The amino acid composition of claim 8 wherein the amino acid comprises from about 5 to about 90 percent of the solids mixture.

15. A method for decreasing viscosity of a protein composition, the method comprising admixing at least one amino acid comprising at least about 90 percent particles of less than about 160 microns with at least one protein to produce a solids mixture comprising the amino acid and protein, suspending the solids mixture in a substantially homogeneous aqueous suspension, and drying the suspension to produce protein composition which is less viscous when reconstituted in aqueous compositions.

## Patentansprüche

1. Verfahren zur Herstellung einer Aminosäurezusammensetzung mit erhöhter Löslichkeit, wobei das Verfahren Folgendes umfasst: Mischen von mindestens einer mikronisierten Aminosäure, die mindestens etwa 90 Prozent Partikel von weniger als etwa 160 Mikron umfasst, mit mindestens einem Protein, um eine Feststoffmischung herzustellen, die die Aminosäure und das Protein umfasst, Suspendieren der Feststoffinischung, um eine im Wesentlichen homogene wässrige Suspension zu bilden, und Trocknen der Suspension, um ein Aminosäure/Protein-Produkt herzustellen.

2. Verfahren nach Anspruch 1, wobei die mikronisierte Aminosäure eine Partikelgröße von etwa 7 bis etwa 10 Mikron aufweist.

3. Verfahren nach Anspruch 1, wobei die Aminosäure eine hydrophobe Aminosäure ist.

4. Verfahren nach Anspruch 1, wobei die Aminosäure eine verzweigtkettige Aminosäure ist.

5. Verfahren nach Anspruch 1, wobei das Protein Peptide umfasst, die von Molkenprotein stammen.

6. Verfahren nach Anspruch 1, wobei die Feststoffmischung von etwa 7 bis etwa 55 Prozent der Suspension umfasst.

7. Verfahren nach Anspruch 1, wobei die Aminosäure von etwa 5 bis etwa 90 Prozent der Feststoffmischung umfasst.

8. Aminosäurezusammensetzung mit verbesserter Löslichkeit von mindestens einer Aminosäure, wobei die Zusammensetzung durch ein Verfahren hergestellt wird, das Folgendes umfasst: gleichzeitiges Verarbeiten einer Mischung, die mindestens eine mikronisierte Aminosäure, die mindestens etwa 90 Prozent Partikel von weniger als etwa 160 Mikron umfasst, und mindestens ein Protein umfasst, um eine im Wesentlichen homogene wässrige Suspension zu bilden; und Trocknen der Suspension, um ein im Wesentlichen festes und/oder pulverisiertes Peptid/Aminosäure-Produkt zu erhalten.

9. Aminosäurezusammensetzung nach Anspruch 8, wobei die mikronisierte Aminosäure eine Partikelgröße von etwa 7 bis etwa 10 Mikron aufweist.

10. Aminosäurezusammensetzung nach Anspruch 8, wobei die Aminosäure eine hydrophobe Aminosäure ist.

11. Aminosäurezusammensetzung nach Anspruch 8, wobei die Aminosäure eine verzweigtkettige Aminosäure ist.

12. Aminosäurezusammensetzung nach Anspruch 8, wobei das Protein Peptide umfasst, die von Molkenprotein stammen.

13. Aminosäurezusammensetzung nach Anspruch 8, wobei die Feststoffmischung von etwa 7 bis etwa 55 Prozent der Suspension umfasst.

14. Aminosäurezusammensetzung nach Anspruch 8, wobei die Aminosäure von etwa 5 bis etwa 90 Prozent der Feststoffmischung umfasst.

15. Verfahren zur Verringerung der Viskosität einer Proteinzusammensetzung, wobei das Verfahren Folgendes umfasst: Mischen von mindestens einer Aminosäure, die mindestens etwa 90 Prozent Partikel von weniger als etwa 160 Mikron umfasst, mit mindestens einem Protein, um eine Feststoffmischung herzustellen, die die Aminosäure und das Protein umfasst, Suspendieren der Feststoffmischung in einer im Wesentlichen homogenen wässrigen Suspension und Trocknen der Suspension, um eine Proteinzusammensetzung herzustellen, die bei Wiederherstellung in wässrigen Zusammensetzungen weniger viskos ist.

## Revendications

1. Procédé de production d'une composition d'acides aminés ayant une solubilité accrue, le procédé comprenant le mélange d'au moins un acide aminé micronisé comprenant au moins 90 pour cent environ de particules de moins de 160 microns environ et d'au moins une protéine pour produire un mélange de solides comprenant l'acide aminé et la protéine, la mise en suspension du mélange de solides pour former une suspension aqueuse substantiellement homogène et le séchage de la suspension pour produire un produit acide aminé/protéine.

2. Procédé selon la revendication 1, où l'acide aminé micronisé a une dimension particulaire qui va de 7 environ à 10 microns environ.

3. Procédé selon la revendication 1, où l'acide aminé est un acide aminé hydrophobe.

4. Procédé selon la revendication 1, où l'acide aminé est un acide aminé à chaîne ramifiée.

5. Procédé selon la revendication 1, où la protéine comprend des peptides dérivés d'une protéine du lactosérum.

6. Procédé selon la revendication 1, où le mélange de solides comprend de 7 environ à 55 pour cent environ de la suspension.

7. Procédé selon la revendication 1, où l'acide aminé comprend de 5 environ à 90 pour cent environ du mélange de solides.

8. Composition d'acides aminés dans laquelle au moins un acide aminé a une solubilité accrue, la composition étant préparée par un procédé qui comprend le co-traitement d'un mélange comprenant au moins un acide aminé micronisé comprenant au moins 90 pour cent environ de particules de moins de 160 microns environ et d'au moins une protéine pour produire une suspension aqueuse substantiellement homogène et le séchage de la suspension pour produire un produit acide aminé/peptide substantiellement solide et/ou pulvérulent.

9. Composition d'acides aminés selon la revendication 8, où l'acide aminé micronisé a une dimension particulaire qui va de 7 environ à 10 microns environ.

10. Composition d'acides aminés selon la revendication 8, où l'acide aminé est un acide aminé hydrophobe.

11. Composition d'acides aminés selon la revendication 8, où l'acide aminé est un acide aminé à chaîne ramifiée.

12. Composition d'acides aminés selon la revendication 8, où la protéine comprend des peptides dérivés d'une protéine du lactosérum.

13. Composition d'acides aminés selon la revendication 8, où le mélange de solides comprend de 7 environ à 55 pour cent environ de la suspension.

14. Composition d'acides aminés selon la revendication 8, où l'acide aminé comprend de 5 environ à 90 pour cent environ du mélange de solides.

15. Procédé permettant de réduire la viscosité d'une composition protéique, le procédé comprenant le mélange d'au moins un acide aminé comprenant au moins environ 90 pour cent de particules de moins de 160 microns environ et d'au moins une protéine pour produire un mélange de solides comprenant l'acide aminé et la protéine, la mise en suspension du mélange de solides pour former une suspension aqueuse substantiellement homogène et le séchage de la suspension pour produire un composition protéique qui est moins visqueuse après reconstitution dans des compositions aqueuses.
